# EUROPEAN PATENT SPECIFICATION

(11) **EP 2 043 451 B1**
(45) Date of publication and mention of the grant of the patent: **21.03.2012**
(21) Application number: 07729993.1
(22) Date of filing: 08.06.2007
(51) Int. Cl.: A23D 9/007, A23L 1/09, A23L 1/22, A23L 1/221, A61Q 13/00

(54) **CYCLODEXTRIN BLENDS WITH CRYSTAL GROWTH INHIBITORS**
CYCLODEXTRINMISCHUNGEN MIT KRISTALLWACHSTUMSHEMMERN
MÉLANGES DE CYCLODEXTRINE AVEC INHIBITEURS DE CROISSANCE DE CRISTAUX

(30) Priority: 18.07.2006 US 807676 P
(43) Date of publication of application: 08.04.2009
(73) Proprietor: Symrise AG, 37603 Holzminden (DE)
(72) Inventor: SIEGEL, Sven, 37671 Höxter (DE)
(74) Representative: Eisenführ, Speiser & Partner
(86) International application number: PCT/EP2007/055634
(87) International publication number: WO 2008/009512

(56) References cited:
- EP-A1- 0 829 531
- WO-A-02/01967
- US-A- 2 413 170
- US-A- 4 532 133
- US-A- 5 280 127
- US-A1- 2002 091 111
- US-A1- 2002 176 879
- US-B1- 6 296 889

## Description

The invention relates to the field of encapsulating aromas with cyclodextrins. In particular, it relates to methods for spray drying aromas to produce aroma/cyclodextrin particles and to the resultant particles themselves.

Aroma/cyclodextrin particles (cyclodextrin particles containing at least one aroma substance) are crystalline substances, which may readily be produced on a small scale by atomization drying methods, in particular spray processes such as spray drying or fluidized bed granulation. Scaling up to production scale is, however, problematic, as unwanted crystal growth occurs with many aromas. Growth may here proceed to such an extent that spray nozzles become clogged. The spray process thus has to be interrupted at certain intervals in order to clean the spray nozzles. This ultimately results in uneconomic production. Moreover, excessively large crystals settle out in containers and piping, so additionally complicating production. The crystals may then, for example, only be incompletely discharged from the preparation vessel and sent for spray drying. Settling in piping results inter alia in disrupted product throughput and makes an additional contribution to spray nozzle clogging. Accumulations of particles may build up which advance slowly towards the spray nozzle and ultimately jointly clog the latter, despite the individual particles being smaller than the nozzle diameter.

### Technical background of the invention

Cyclodextrins are oligomers of anhydroglucose building blocks which are linked together via alpha-1,4 bonds to form an annular molecule. Depending on the number of building blocks, it is possible to differentiate alpha- (6 building blocks), beta- (7 building blocks) and gamma- (8 building blocks) cyclodextrins. These are conventionally produced from starch by enzymatic methods. The cyclic structure of cyclodextrins permits the formation of inclusion complexes from a cyclodextrin molecule and an included guest substance on a molecular plane. Depending on the geometry and polarity of the aroma substances, the inclusion compounds may be formed more or less completely.

Aroma substances are usually volatile compounds which suffer high levels of loss due to vaporization or oxidation during storage or also during processing for example in the food industry. Aroma substances may be protected from such loss in cyclodextrin complexes.

Aroma substances or aromas generally comprise volatile, usually liquid substances or complex blends of these substances.

Various methods are known for the production of inclusion compounds of aroma substances with cyclodextrins.

These methods involve mixing cyclodextrin solutions, suspensions, or pastes (conventionally in water) and the aroma substances. Depending on the polarity of the aroma substances, this usually gives rise to a two-phase mixture. Elevated shear forces, applied for example by stirring or kneading, are accordingly used to accelerate the method. This stage is usually followed by a drying step, such as for example spray, freeze, or fluidized bed drying.

According to the known prior art, very fine particles are frequently obtained when drying aqueous aroma/cyclodextrin complexes by spraying.

EP 0 392 608 (which corresponds to US 5,635,238) describes a method for the production of pulverulent cyclodextrin complexes, the particle size of which is below 12 µm, preferably below 5 µm. Processes such as for example spray drying and freeze drying are used here.

WO 01/48024 describes the production of cyclodextrin complexes (cyclodextrin/guest complexes, wherein the guest may be, for example, an odoriferous or aroma substance) with the assistance of an emulsifying agent. Complexes of beta- or gamma-cyclodextrin and fish oil (containing omega-3 fatty acids) were produced, inter alia. These cyclodextrin complexes may be converted into a dry form (powder) inter alia by means of spray drying. No details are stated regarding batch size.

WO 02/49455 describes the use of cyclodextrin complexes for the preparation of foodstuffs. Complexes of cyclodextrins and strawberry or onion aroma were explicitly subjected to organoleptic investigation. Aroma encapsulation may here proceed by coprecipitation followed by spray drying. No details are stated regarding batch size during production of the complexes.

EP 1 084 625 describes the production of cyclodextrin complexes with the assistance of solids content-increasing agents, such as gum arabic, maltodextrin or modified starches, these preferably being dried by means of spray drying. The inclusion of a tomato aroma in beta-cyclodextrin was explicitly described, the respective content of the aroma substances dimethyl sulfoxide, cis-3-hexenol, 2-isobutylthiazole, phenylethyl alcohol and beta-damascenone in the aroma/cyclodextrin complex being determined chromatographically. In addition to tomato aroma, aromas of the shrimp, beef and chicken flavor directions were also complexed with beta-cyclodextrin. The aroma substances dimethyl sulfide, methylmercaptan, acetaldehyde, 2-methyl-3-furanthiol, diacetyl or aromas produced by means of pyrolysis are preferably encapsulated according to EP 1 084 625. No details are stated regarding batch size during production of the complexes.

EP 1 084 625 furthermore acknowledges numerous items of patent literature relating to cyclodextrin complexes. Reference may in particular be made at this point to various US patents in connection with aroma/cyclodextrin complexes: US 4,024,223 (menthol, methyl salicylate), US 4,474,822 (tea essences), US 4,529,608 (honey aroma), US 4,560,571 (coffee extract, instant beverages), US 4,001,438 (peppermint aroma, chewing gum), US 3,061,444 (meat and vegetable aromas) or US 5,571,782 (numerous aroma substances).

CN 1073711 1 describes blends comprising beta-cyclodextrin, carboxymethylcellulose and essences or perfumes, such as for example jasmine, rose or lily of the valley, which may for example be converted into granular form by means of spray drying. No details are stated regarding batch size during production of the complexes.

Specific complexes of cyclodextrins and essential fatty acids which comprise at least two double bonds, such as linoleic acid, linolenic acid or arachidonic acid, are produced in EP 1 419 761. Only laboratory scale batch sizes were described.

EP 1 537 174 describes cellulose ether-containing cyclodextrin particles which contain aroma and/or odoriferous substances with a particle size in the range from 50 to 1000 µm. Aroma/cyclodextrin complexes which are explicitly described therein comprise, for example, d-limonene, peppermint aroma, beef aroma, lemon oil or cis-3-hexenol. The maximum batch size was 18 kg.

The Journal of Inclusion Phenomena and Molecular Recognition in Chemistry 1995, 23, 11-21 investigated, on the one hand, the effects of cosolvents on the solubility of beta-cyclodextrin in aqueous systems and, on the other hand, the selectivity of formation of the inclusion complexes of beta-cyclodextrin with the odoriferous and aroma substances pinene, eugenol, cineole, limonene and camphor. The cosolvents used were methanol, ethanol, isopropanol, acetonitrile, tetrahydrofuran, ethylene glycol dimethyl ether, acetone, dioxane, dimethylformamide, dimethyl sulfoxide, pyridine, ethylene glycol and formamide.

Biosci. Biotechnol. Biochem. 1998, 62(11), 2166-2170 describes the formation of inclusion complexes from alpha-, beta- or gamma-cyclodextrin and d-limonene, phenylethyl alcohol, acetophenone or menthol in various organic (aqueous) solvents. The solvents used here were ethanol, methanol, acetonitrile, isopropanol, n-propanol, toluene, hexane, isopropyl ether, acetone, ethyl acetate, methyl ethyl ketone, tetrahydrofuran, diethyl ether, 1,4-dioxane, 1-butanol and ethylene glycol dimethyl ether. The best molar inclusion ratios of d-limonene in beta-cyclodextrin were here obtained in (aqueous) ethanol or methanol.

The effect of water and alcohol on the formation of inclusion complexes of d-limonene and cyclodextrin is also described in Supramolecular Chem. 1993, 1, 321-325.

J. Agric. Food. Chem. 2005, 53, 388-392 describes the influence of solvent on inclusion complexes of aroma substances and alpha-, beta- or gamma-cyclodextrin. The aroma substances investigated therein were ethyl butyrate, ethyl heptanoate, citral (therein as a 1:1 blend of neral and geranial), I-menthol and methyl anthranilate, and the (aqueous) solvents were ethanol, propylene glycol, triacetin (glycerol triacetate) and triethyl citrate.

The prior art provides no description of how cyclodextrin complexes may be produced on a larger (industrial) scale and how the above-stated problems which arise in so doing may be solved.

### Description

The production of aroma substance/cyclodextrin inclusion compounds conventionally proceeds by vigorous mixing of the aroma substances with aqueous cyclodextrin solutions, suspensions, or pastes. Elevated shear forces, applied for example by stirring or kneading, are accordingly used to accelerate the method. This stage is usually followed by a drying step. Spray drying or fluidized bed spray granulation methods are frequently used due to their elevated efficiency.

The production of aroma/cyclodextrin particles on a small scale is in principle unproblematic. However, when larger quantities are produced, the surface area to volume ratio becomes smaller. Consequently, the mixing energy which is converted by dissipation into thermal energy may be less readily released into the surrounding environment and the spray blend is subjected to strong heating. The elevated temperature leads to increased solubility of the cyclodextrin complex. The spray blend cools while it is being conveyed through piping, as a result of which solubility falls and crystal growth starts. The crystals may grow to a size of several millimeters and finally clog the spray nozzles. This crystal growth results in reduced throughput, frequent cleaning of the nozzles and may even make production impossible. Crystal growth in particular occurs when an aroma outside the group of spices and fruit aromas is to be encapsulated in cyclodextrin.

It was therefore the object of the invention to provide an atomization drying method and preferably a spray drying method for encapsulating aromas in which the above disadvantages of conventional encapsulation methods, in particular spray drying methods, are reduced or eliminated. It was further intended to provide correspondingly encapsulatable and preferably spray dryable blends and correspondingly encapsulated, preferably spray dried products. The intention was that the flavor of encapsulated aromas should be deteriorated as little as possible.

Said object is achieved by the use of a terpene of the empirical formula C₁₀H₁₆ and/or a mono- or polyunsaturated linear fatty acid having 16 to 18 carbon atoms as a crystal growth inhibitor, in particular in atomization drying methods. Said use includes the use of (i) blends of terpenes and (ii) blends of fatty acids and (iii) blends of terpenes and fatty acids. The stated terpenes and/or fatty acids are used for encapsulating in particular crystal growth-promoting aroma substances in cyclodextrin particles.

Thanks to the use according to the invention, it is for the first time straightforwardly possible to encapsulate aromas or aroma blends containing crystal growth-promoting aroma substances with cyclodextrins in industrially significant batches by atomization drying methods (see below for further details), so reducing or completely eliminating the disadvantages of the prior art. Thanks to the use according to the invention of terpenes of the empirical formula C₁₀H₁₆ and/or mono- or polyunsaturated linear fatty acids having 16 to 18 carbon atoms, cyclodextrin/aroma complexes may successfully be spray-granulated in trouble-free manner with a continuous throughput of at least 50 l, preferably at least 100 l and particularly preferably of 100 to 500 l of spray blend per atomizer. An atomizer is here taken to mean a device for producing a mist or aerosol, as is conventional in atomization drying and in particular in spray drying. In particular, a nozzle or atomizing disk conventionally used for spray drying is an atomizer for the purposes of the invention. Suitable atomizers and spray drying methods are described below in greater detail. The use according to the invention thus makes it possible continuously to spray dry even a large volume of 50 l or more of a spray blend which is to be spray dried with an individual atomizer, without its being necessary, as hitherto, to divide the volume to be spray dried into smaller individual volumes to be spray dried or to interrupt the spray drying operation in order to clean undesirably large crystals from the apparatus used for spray drying.

The use according to the invention is particularly worthwhile for atomization drying, in particular spray drying, crystal growth promoting aroma substances. For the purposes of the present invention, the following are crystal growth-promoting aroma substances: 2-methyl-3-(methylthio)furan, 2-methyl-3-furanthiol, bis(2-methyl-3-furyl) disulfide, furfurylmercaptan, methional, 2-acetyl-2-thiazoline, 3-mercapto-2-pentanone, 2,5-dimethyl-3-furanthiol, 2,4,5-trimethylthiazole, 2-acetylthiazole, 2,4-dimethyl-5-ethylthiazole, mercapto-3-methyl-1-butanol, 2-acetyl-1-pyrroline, 2-methyl-3-ethylpyrazine, 2-ethyl-3,5-dimethylpyrazine, 2-ethyl-3,6-dimethylpyrazine, 2,3-diethyl-5-methylpyrazine, 3-isopropyl-2-methoxypyrazine, 3-isobutyl-2-methoxypyrazine, 2-acetylpyrazine, 2-pentylpyridine, (E,E)-2,4-decadienal, (E,E)-2,4-nonadienal, (E)-2-octenal, (E)-2-nonenal, 2-undecenal, 12-methyltridecanal, 1-penten-3-one, diacetyl (= 2,3-butanedione), 4-hydroxy-2,5-dimethyl-3(2H)-furanone, guaiacol, 3-hydroxy-4,5-dimethyl-2(5H)-furanone, 3-hydroxy-4-methyl-5-ethyl-2(5H)-furanone, dimethyl sulfide and trimethylamine, and blends containing two or more of the stated aroma substances.

The crystal growth-promoting aroma substances may for example be a constituent of aromas of the following flavor directions: meat (for example pork, beef, chicken/poultry aromas); roast/baked products (for example coffee, cocoa, chocolate, bread, roast potato aromas); milk products (for example cheese, milk, yogurt, caramel aromas); seafood or fish.

Preferred crystal growth inhibitors for the purposes of the present invention are substances which, in addition to the above-stated conditions, exhibit a slight intrinsic flavor, so that they have little or no influence on the flavor of the aroma to be encapsulated. This is in particular the case for substances having a flavor threshold value of greater than 100 ppb (measured in water). The higher the flavor threshold value, the greater is the quantity of the corresponding substance which must be used for it to be perceived in flavor terms. Since it is intended for the crystal growth inhibitor as far as possible to be imperceptible or only slightly perceptible in flavor terms, crystal growth inhibitors with comparatively elevated flavor threshold values, in the present case thus greater than 100 ppb (measured in water), are to be preferred.

The crystal growth inhibitors to be used according to the invention do not per se prevent the formation of solid particles in a spray solution according to the invention, but instead bring about a small solid particle size, whereby clogging of the spray nozzles can be avoided.

The terpenes (i) are preferably selected from the group consisting of limonene, alpha-pinene, beta-pinene, alpha-terpinene, gamma-terpinene, terpinolene, alpha-phellandrene, beta-phellandrene and carene. Preferred crystal growth inhibitors among the terpenes are d-limonene, I-limonene, racemic limonene (dipentene), alpha-pinene (preferably d-, I- or any desired mixture thereof), delta-carene, with d-limonene being particularly preferred.

The mono- or polyunsaturated linear fatty acids having 16 to 18 carbon atoms (ii) preferably comprise mono-, di- or triunsaturated linear fatty acids having 16, 17 or 18 carbon atoms, preferably palmitoleic acid (cis-9-hexadecenoic acid), oleic acid (cis-9-octadecenoic acid), linoleic acid (cis,cis-octadeca-9,12-dienoic acid) and linolenic acid (for example gamma-linolenic acid, (all-cis)-octadeca-6,9,12-trienoic acid), with oleic acid (cis-9-octadecenoic acid) being preferred.

Cyclodextrins which are suitable according to the invention are alpha-, beta-, gamma- and substituted cyclodextrins. Alpha-, beta-, gamma-cyclodextrin or mixtures thereof are advantageous, with beta-cyclodextrin being preferred. In a preferred embodiment, the cyclodextrin fraction of the spray blend consists solely of beta-cyclodextrin.

Cyclodextrin particles are furthermore stated according to the invention which contain or consist of:
(a) 70-99.3 wt.%, preferably 80-96.7 wt.%, of one or more cyclodextrins,
(b) 0.001-20 wt.%, preferably 0.01-10 wt.% and particularly preferably 0.01-5 wt.%, of a crystal growth-promoting aroma substance selected from the group consisting of 2-methyl-3-(methylthio)furan, 2-methyl-3-furanthiol, bis(2-methyl-3-furyl) disulfide, furfurylmercaptan, methional, 2-acetyl-2-thiazoline, 3-mercapto-2-pentanone, 2,5-dimethyl-3-furanthiol, 2,4,5-trimethylthiazole, 2-acetylthiazole, 2,4-dimethyl-5-ethylthiazole, mercapto-3-methyl-1-butanol, 2-acetyl-1-pyrroline, 2-methyl-3-ethylpyrazine, 2-ethyl-3,5-dimethylpyrazine, 2-ethyl-3,6-dimethylpyrazine, 2,3-diethyl-5-methylpyrazine, 3-isopropyl-2-methoxypyrazine, 3-isobutyl-2-methoxypyrazine, 2-acetylpyrazine, 2-pentylpyridine, (E,E)-2,4-decadienal, (E,E)-2,4-nonadienal, (E)-2-octenal, (E)-2-nonenal, 2-undecenal, 12-methyltridecanal, 1-penten-3-one, 2,3-butanedione, 4-hydroxy-2,5-dimethyl-3(2H)-furanone, guaiacol, 3-hydroxy-4,5-dimethyl-2(5H)-furanone, 3-hydroxy-4-methyl-5-ethyl-2(5H)-furanone, dimethyl sulfide and trimethylamine, or a blend of two or more of these aroma substances,
(c) 0.1-2 wt.%, preferably 0.2-1.5 wt.% and particularly preferably 0.3-1 wt.%, of a crystal growth inhibitor selected from the group consisting of
   (i) terpenes of the empirical formula C₁₀H₁₆ and
   (ii) mono- or polyunsaturated linear fatty acids having 16 to 18 carbon atoms,
   in each case relative to the total weight of the aroma particles, preferably furthermore
(d) 0.5-14 wt.% of water, preferably 3-10 wt.% of water,
   and optionally
(e) 0-14 wt.% particularly preferably 0-7 wt.%, very particularly preferably 0-5 wt.% of one or more aroma solvents, and/or
(f) one or more further aroma substances up to a total proportion of the aroma substances according to (b) and (f) of preferably up to 20 wt.%.

For the purposes of the invention, aroma solvents are solvents which are suitable for human consumption at least for a crystal growth-promoting aroma substance and preferably also for the further aroma substance(s). Preferred aroma solvents are ethanol, fatty oils, for example edible oils and in particular vegetable oils such as for example borage oil, thistle oil, peanut oil, hazelnut oil, coconut oil, pumpkin seed oil, linseed oil, maize germ oil, macadamia oil, almond oil, olive oil, pecan oil, pistachio oil, rapeseed oil, rice germ oil, sesame oil, soya oil, sunflower oil, walnut oil or wheat germ oil, with fractionated coconut oils which primarily comprise fatty acid residues with a length of six to eight carbon atoms (C6-C8 fatty acids) being preferred, propylene glycol, diacetin (glycerol diacetate), triacetin (glycerol triacetate), benzyl alcohol, triethyl citrate, ethyl lactate, isopropanol and glycerol.

Preferred cyclodextrin particles accordingly contain or consist of:
(a) 70-99.3 wt.% of β-cyclodextrin,
(b) 0.01-10 wt.% and particularly preferably 0.01-5 wt.% of a crystal growth promoting aroma substance selected from the group consisting of 2-methyl-3-(methylthio)furan, 2-methyl-3-furanthiol, bis(2-methyl-3-furyl) disulfide, furfurylmercaptan, methional, 2-acetyl-2-thiazoline, 3-mercapto-2-pentanone, 2,5-dimethyl-3-furanthiol, 2,4,5-trimethylthiazole, 2-acetylthiazole, 2,4-dimethyl-5-ethylthiazole, mercapto-3-methyl-1-butanol, 2-acetyl-1-pyrroline, 2-methyl-3-ethylpyrazine, 2-ethyl-3,5-dimethylpyrazine, 2-ethyl-3,6-dimethylpyrazine, 2,3-diethyl-5-methylpyrazine, 3-isopropyl-2-methoxypyrazine, 3-isobutyl-2-methoxypyrazine, 2-acetylpyrazine, 2-pentylpyridine, (E,E)-2,4-decadienal, (E,E)-2,4-nonadienal, (E)-2-octenal, (E)-2-nonenal, 2-undecenal, 12-methyltridecanal, 1-penten-3-one, 2,3-butanedione, 4-hydroxy-2,5-dimethyl-3(2H)-furanone, guaiacol, 3-hydroxy-4,5-dimethyl-2(5H)-furanone, 3-hydroxy-4-methyl-5-ethyl-2(5H)-furanone, dimethyl sulfide and trimethylamine, or a blend of two or more of these aroma substances,
(c) 0.2-1.5 wt.% and particularly preferably 0.3-1 wt.% of a crystal growth inhibitor selected from the group consisting of
   (i) limonene, alpha-pinene, beta-pinene, alpha-terpinene, gamma-terpinene, terpinolene, alpha-phellandrene, beta-phellandrene and carene. Preferred crystal growth inhibitors among the terpenes are d-limonene, racemic limonene (dipentene), alpha-pinene (preferably d-, I- or any desired mixture thereof), delta-carene, with d-limonene being particularly preferred,
   (ii) palmitoleic acid (cis-9-hexadecenoic acid), oleic acid (cis-9-octadecenoic acid), linoleic acid (cis,cis-octadeca-9,12-dienoic acid) and linolenic acid (for example gamma-linolenic acid, (all-cis)-octadeca-6,9,12-trienoic acid), with oleic acid (cis-9-octadecenoic acid) being preferred
   or blends of two or more of these substances,
(d) 0.5-14 wt.%, preferably 3-10 wt.%, of water,
   and optionally
(e) 0-14 wt.%, particularly preferably 0-7 wt.%, very particularly preferably 0-5 wt.% of an aroma solvent selected from the group consisting of ethanol, fatty oils, for example edible oils and in particular vegetable oils such as for example borage oil, thistle oil, peanut oil, hazelnut oil, coconut oil, pumpkin seed oil, linseed oil, maize germ oil, macadamia oil, almond oil, olive oil, pecan oil, pistachio oil, rapeseed oil, rice germ oil, sesame oil, soya oil, sunflower oil, walnut oil or wheat germ oil, with fractionated coconut oils which primarily comprise fatty acid residues with a length of six to eight carbon atoms (C6-C8 fatty acids) being preferred, propylene glycol, diacetin (glycerol diacetate), triacetin (glycerol triacetate), benzyl alcohol, triethyl citrate, ethyl lactate, isopropanol and glycerol, or blends of two or more of these aroma solvents, and optionally
(f) one or more further aroma substances,
wherein the weight percentages are in each case relative to the total weight of the aroma particles, and the preferred cyclodextrin particle contains in total up to 20 wt.% of aroma substances (crystal growth-promoting aroma substances (b) and one or more further aroma substances (f)).

The aroma-containing cyclodextrin particles according to the invention are produced by initially producing an aqueous spray blend containing the aroma(s) to be spray dried, optionally in an aroma solvent, the cyclodextrin(s), and the crystal growth inhibitor(s); see below for further details. The aroma-containing cyclodextrin particles according to the invention are produced from this spray blend by means of an atomization drying method, in particular a spraying process.

Spray blends which are preferred according to the invention contain or consist of:
(a) 5-40 wt.%, preferably 15-30 wt.%, of one or more cyclodextrins,
(b) 0.0001-8 wt.%, preferably 0.003-4 wt.% of a crystal growth-promoting aroma substance selected from the group consisting of 2-methyl-3-(methylthio)furan, 2-methyl-3-furanthiol, bis(2-methyl-3-furyl) disulfide, furfurylmercaptan, methional, 2-acetyl-2-thiazoline, 3-mercapto-2-pentanone, 2,5-dimethyl-3-furanthiol, 2,4,5-trimethylthiazole, 2-acetylthiazole, 2,4-dimethyl-5-ethylthiazole, mercapto-3-methyl-1-butanol, 2-acetyl-1-pyrroline, 2-methyl-3-ethylpyrazine, 2-ethyl-3,5-dimethylpyrazine, 2-ethyl-3,6-dimethylpyrazine, 2,3-diethyl-5-methylpyrazine, 3-isopropyl-2-methoxypyrazine, 3-isobutyl-2-methoxypyrazine, 2-acetylpyrazine, 2-pentylpyridine, (E,E)-2,4-decadienal, (E,E)-2,4-nonadienal, (E)-2-octenal, (E)-2-nonenal, 2-undecenal, 12-methyltridecanal, 1-penten-3-one, 2,3-butanedione, 4-hydroxy-2,5-dimethyl-3(2H)-furanone, guaiacol, 3-hydroxy-4,5-dimethyl-2(5H)-furanone, 3-hydroxy-4-methyl-5-ethyl-2(5H)-furanone, dimethyl sulfide and trimethylamine, or a blend of two or more of these aroma substances,
(c) 0.07-1.5 wt.%, preferably 0.14-1 wt.% and particularly preferably 0.2-0.7 wt.%, of a crystal growth inhibitor selected from the group consisting of
   (i) terpenes of the empirical formula C₁₀H₁₆ and
   (ii) mono- or polyunsaturated linear fatty acids having 16 to 18 carbon atoms,
   and blends of two or more of the crystal growth inhibitors,
(d) 60-94.8 wt.%, preferably 70-84.6 wt.%, of water,
   and optionally
(e) 0-6 wt.%, preferably 0-3 wt.%, of one or more aroma solvents as described above, and optionally
(f) one or more further aroma substances up to a total proportion of the aroma substances according to (b) and (f) of preferably up to 20 wt.%, in each case relative to the total weight of the spray blend.

A preferred spray blend is in particular one which contains or consists of:
(a) 15-30 wt.% of β-cyclodextrin,
(b) 0.003-4 wt.% of a crystal growth-promoting aroma substance selected from the group consisting of 2-methyl-3-(methylthio)furan, 2-methyl-3-furanthiol, bis(2-methyl-3-furyl) disulfide, furfurylmercaptan, methional, 2-acetyl-2-thiazoline, 3-mercapto-2-pentanone, 2,5-dimethyl-3-furanthiol, 2,4,5-trimethylthiazole, 2-acetylthiazole, 2,4-dimethyl-5-ethylthiazole, mercapto-3-methyl-1-butanol, 2-acetyl-1-pyrroline, 2-methyl-3-ethylpyrazine, 2-ethyl-3,5-dimethylpyrazine, 2-ethyl-3,6-dimethylpyrazine, 2,3-diethyl-5-methylpyrazine, 3-isopropyl-2-methoxypyrazine, 3-isobutyl-2-methoxypyrazine, 2-acetylpyrazine, 2-pentylpyridine, (E,E)-2,4-decadienal, (E,E)-2,4-nonadienal, (E)-2-octenal, (E)-2-nonenal, 2-undecenal, 12-methyltridecanal, 1-penten-3-one, 2,3-butanedione, 4-hydroxy-2,5-dimethyl-3(2H)-furanone, guaiacol, 3-hydroxy-4,5-dimethyl-2(5H)-furanone, 3-hydroxy-4-methyl-5-ethyl-2(5H)-furanone, dimethyl sulfide and trimethylamine, or a blend of two or more of these aroma substances,
(c) 0.14-1 wt.% and particularly preferably 0.2-0.7 wt.%, of a crystal growth inhibitor selected from the group consisting of
   (i) terpenes of the empirical formula C₁₀H₁₆ and
   (ii) mono- or polyunsaturated linear fatty acids having 16 to 18 carbon atoms,
   or blends two or more of the crystal growth inhibitors,
(d) 70-84.6 wt.% of water,
   and optionally
(e) 0-6 wt.%, preferably 0-3 wt.%, of an aroma solvent selected from the group consisting of ethanol, fatty oils, for example edible oils and in particular vegetable oils such as for example borage oil, thistle oil, peanut oil, hazelnut oil, coconut oil, pumpkin seed oil, linseed oil, maize germ oil, macadamia oil, almond oil, olive oil, pecan oil, pistachio oil, rapeseed oil, rice germ oil, sesame oil, soya oil, sunflower oil, walnut oil or wheat germ oil, with fractionated coconut oils which primarily comprise fatty acid residue with a length of six to eight carbon atoms (C6-C8 fatty acids) being preferred, propylene glycol, diacetin (glycerol diacetate), triacetin (glycerol triacetate), benzyl alcohol, triethyl citrate, ethyl lactate, isopropanol and glycerol, or blends of two or more of these aroma solvents, and optionally (f) one or more further aroma substances up to a total proportion of the aroma substances according to (b) and (f) of preferably up to 15 wt.%, in each case relative to the total weight of the spray blend.

Thanks to the addition according to the invention of one or more crystal growth inhibitors (constituent (c) of a spray blend according to the invention), the formation of solid particles with a size of greater than 250 micrometers, which would clog the spray nozzles, is completely or extensively prevented in a spray blend according to the invention.

In preferred spray blends according to the invention, the size of the solid particles is less than or equal to 125 micrometers, particularly preferably less than 50 micrometers, especially preferably less than or equal to 30 micrometers.

Particle size determination via volume distribution may proceed by means of laser diffraction (for example using the Master Sizer^{®} MSS Longbench, or the Mastersizer Micro from Malvern Instruments Ltd., Malvern, UK). Since it is of particular significance in this invention to obtain information regarding the size of the large particles, it is recommended to use the D (v, 0.9) value. The D(v, 0.9) value indicates that particle size x which, in a volume-based cumulative distribution function Q₃ (x), is assigned to the 0.9 fraction. Unless otherwise stated, the stated particle size values relate to the D(v, 0.9) value. Particle size may also be determined by measurement under a microscope. This is, for example, advisable if, due to particles having been preselected, it is not appropriate to prepare a cumulative distribution function. This is, for example, the case if crystal sizes in a clogged spray nozzle are being investigated.

Moreover, a spray blend according to the invention or a cyclodextrin particle according to the invention may contain one or more additives or auxiliary substances, such as for example emulsifiers according to WO 01/48024 and/or solids content-increasing agents according to EP 1 084 625.

Additives or auxiliary substances which the spray blend may, for example, contain are binders in the form of cellulose ethers, as described in EP 1 537 174. Preferred cellulose ethers are selected from the group comprising methylcellulose, ethylcellulose, propylcellulose, hydroxyethylcellulose, hydroxypropylcellulose, hydroxypropylmethylcellulose, carboxymethylcellulose, carboxymethylhydroxyethylcellulose and ethylhydroxyethylcellulose, with carboxymethylcellulose (CMC) being particularly preferred.

Particularly preferred additives or auxiliary substances are: maltodextrins, glucose syrup, gum arabic, octenylsuccinated starch (starch sodium octenylsuccinate (E1450)), carboxymethylcellulose, methylcellulose, starch, modified starch.

The total proportion of the additives or auxiliary substances may be in the range from 0 to 50 wt.%, relative to the total weight of a cyclodextrin particle according to the invention or in the range from 0 to 25 wt.%, relative to the total weight of a spray blend according to the invention.

The cyclodextrin particles according to the invention may furthermore contain nutritionally active substances or mixtures of substances (nutraceuticals). The following may, for example be mentioned: panthenol, pantothenic acid, essential fatty acids, vitamin A and derivatives, carotene, vitamin C (ascorbic acid), vitamin E (tocopherol) and derivatives, vitamins of the B and D series, such as vitamin B₆ (nicotinamide), vitamin B₁₂, vitamin D₁, vitamin D₃, vitamin F, folic acid, biotin, amino acids, compounds of the elements magnesium, silicon, phosphorus, calcium, manganese, iron or copper, coenzyme Q10, unsaturated fatty acids, polyunsaturated fatty acids, eicosapentaenoic acid, docosahexaenoic acid and the derivatives thereof, bisabolol, chloramphenicol, caffeine, capsaicin, prostaglandins, thymol, camphor, extracts or other products of plant and animal origin, for example evening primrose oil, borage oil or blackcurrant pip oil, fish oils, fish-liver oil, ceramides and ceramide-like compounds. Plant extracts such as for example arnica, aloe, beard lichen, ivy, stinging nettle, ginseng, henna, chamomile, marigold, rosemary, sage, horsetail or thyme. Oils such as apricot kernel oil, avocado oil, babassu oil, cottonseed oil, borage oil, thistle oil, peanut oil, gamma-oryzanol, rose hip seed oil, hemp oil, hazelnut oil, blackcurrant seed oil, jojoba oil, cherry stone oil, salmon oil, linseed oil, maize germ oil, macadamia oil, almond oil, evening primrose oil, mink oil, olive oil, pecan oil, peach stone oil, pistachio oil, rapeseed oil, rice germ oil, castor oil, safflower oil, sesame oil, soya oil, sunflower oil, tea tree oil, grapeseed oil or wheat germ oil.

It is, of course, possible for the cyclodextrin particles according to the invention to contain still further substances, such as for example emulsifiers, dyes, antioxidants, stabilizers, UV filters, vitamins and other constituents conventional in the foodstuffs, personal hygiene, pharmaceuticals or odoriferous substance industries.

The cyclodextrin particles according to the invention are produced by vigorous mixing of cyclodextrin, water, optionally aroma solvent and at least one crystal growth-promoting aroma substance, in the presence of at least one crystal growth inhibitor to be used according to the invention. Mixing is here carried on until the crystal growth-promoting aroma substances, the crystal growth inhibitors and optionally the aroma solvent have formed inclusion compounds with the cyclodextrin and the equilibrium has shifted towards the inclusion compounds. Performance of the mixing process is known to a person skilled in the art and may, for example, be carried out with stirrers, rotor-stator dispersers, high pressure homogenizers, ultrasound sonotrodes etc..

The spray blend may, for example, be produced as explained in EP-A 1 084 625. In particular, the spray blend may be produced as described below:
A complex is formed from cyclodextrin, the aroma comprising one or more crystal growth-promoting aroma substances and one or more crystal growth inhibitors. The stated starting materials at least partially dissolved for this purpose. The solvent used for this purpose is water at a temperature in the range from 10 to 90°C, preferably in the range from 20 to 70°C, particularly preferably in the range from 20 to 60°C. Temperatures in the range from 15 to 30°C are preferred for aroma substances having a particularly low boiling point or elevated temperature sensitivity.

Depending on the polarity of the aroma substances, a multiphase mixture is generally obtained at this point. According to the invention, shear forces are applied in order to accelerate the method. The shear forces may, for example, be applied by stirring or dispersing, with rotor-stator dispersing tools or high pressure homogenizers inter alia being suitable for this purpose. The duration of the mixing process depends on the tool which is used and the particular aroma which is to be encapsulated. The mixing time is, for example, between 30 seconds and 24 hours. It is preferably between 3 minutes and 4 hours and particularly preferably between 5 and 20 minutes.

After the mixing process, it may be necessary to leave the batch to stand for a certain time in order to shift the equilibrium state as far as possible towards the complex. Conventional standing times range from 0 min to 24 hours and preferably from 1-16 hours. It may here be advantageous to cool the spray blend to a temperature below 25°C, it being entirely advisable to reduce the temperature to 4-7°C in the case of readily soluble complexes.

Complexation of the crystal growth-promoting aromas preferably proceeds in the presence of at least one crystal growth inhibitor. It is, however, also possible, prior to complexation of the crystal growth-promoting aromas, to mix at least one crystal growth inhibitor with the cyclodextrin and to complex it at least partially to completely with the cyclodextrin. These two variants may be considered for preventive suppression of any crystal growth. If crystal growth has already occurred, the crystal growth inhibitor(s) may still also be added after complexation of the aroma and complexed with the cyclodextrin in order to stop and optionally reduce crystal growth.

The spray blend is then dried in an atomization drying method, preferably in a spray process. Drying preferably proceeds by spray drying, fluidized bed spray granulation, rotor granulation, spray drying with an integral fluidized bed or spray drying with a downstream fluidized bed. Any desired type of atomizer may be used for this purpose, such as for example nozzles, mechanical atomizers and ultrasound atomizers. Nozzles which may, for example, be used are single-fluid nozzles, pressure nozzles, solid cone pressure nozzles, hollow cone spray nozzles, two-fluid nozzles, three-fluid nozzles and four-fluid nozzles etc.. Mechanical atomizers (rotary atomizers) comprise a rotating body which breaks the spray blend up into small drops. The rotating bodies are conventionally disks or also bells; a disk atomizer is one example of a mechanical atomizer. Suitable atomizers may be selected by a person skilled in the art in particular depending on quantity of spray blend to be dried and the size of the anticipated aroma particles. Nozzles are preferably used as the atomizer. In particular, nozzles having a bore diameter of 0.3-7 mm may be used. Nozzle having a bore diameter of 0.5-3 mm are preferably used. In the case of pressure nozzles with an annular gap, these values apply analogously to the annular gap. In the case of spring-actuated pressure nozzles, the annular gap varies depending on the applied pressure, such that the annular gap may also be of smaller dimensions than 0.5 mm.

The temperatures at which the respective drying systems are used are conventionally 100-350°C at the air inlet and 50-150°C at the air outlet. Preferably, air inlet temperatures are 130-220°C and air outlet temperatures 50-100°C. Depending on the nature of the aroma to be encapsulated (crystal growth promoting aroma substance and optionally one or more further aroma substances), a person skilled in the art may, however, select a different temperature.

Depending on the method used and the selected method parameters, dried cyclodextrin particles are obtained with a particle size D(v; 0.5) of approx. 1-5000 µm, the range between 20 and 2000 µm being preferred.

Examples of fluidized bed spray granulation are continuous fluidized bed spray granulation according to EP-A 163 836 and discontinuous fluidized bed spray granulation, for example according to EP-A 70 719. Examples of rotor granulation are described in WO-A 97/16078 for the production of aroma granules in a conventional discontinuously operated fluidized bed rotor granulator.

The cyclodextrin particles according to the invention may be used in a plurality of products. In foodstuffs and products consumed for pleasure, they may, for example, be used in confectionery such as for example hard candies, chewing gum, sugar-coated refreshing lozenges, compressed lozenges, hard caramels, candies and chocolate, bakery products such as cakes, wafers and cookies, snacks, instant meals and other instant products (soups, sauces, beverage powders and granules, tea bags, seasoning mixtures). The cyclodextrin particles are particularly suitable for foodstuffs which are heated, superheated, kept warm, sterilized or pasteurizable, such as for example catering foodstuffs, fried foodstuffs, chips, flavored breadcrumb coatings, ready meals, microwave meals and preserved fruit and vegetables.

Pharmaceutical products may, for example, comprise suckable tablets, throat or cough candies, pharmaceutical powders or granules.

Consumer articles may, for example, comprise personal hygiene products, household products, tobacco products (for example cigarettes), cosmetics, detergents and cleaning agents, air fresheners, textiles or odor-absorbing preparations (for example cat litter).

Personal hygiene products may, for example, comprise oral care products such as toothpastes, tooth gels, tooth creams, dental care chewing gums and mouthwashes. The object is achieved by a method for producing aroma particles comprising spray drying a spray blend according to claim 4 through an atomizer.

The following Examples illustrate the present invention without consequently limiting the scope of protection of the claims.

### Examples

Unless otherwise stated, all values stated relate to the total weight of the particular preparation.

### Example 1

Spray blend:
68.90 wt.% of water
28.00 wt.% of beta-cyclodextrin
2.80 wt.% of aroma
0.28 wt.% of d-limonene (crystal growth inhibitor)

Proportions of crystal growth promoting aroma substances in the aroma used:

| Aroma | Proportion of crystal growth promoting aroma substances in the particular aroma |
|---|---|
| Chicken | 43.4 wt.% of (E,E)-2,4-decadienal |
| | 0.3 wt.% of 2-methyl-3-furanthiol |
| Beef | 0.4 wt.% of 2-methyl-3-furanthiol |
| | 1.1 wt.% of methional |
| Roast potato | 2.4 wt.% of 2-ethyl-3,5-dimethylpyrazine |
| | 1.4 wt.% of methional |

In addition to the crystal growth-promoting aroma substances, the aromas also contain one or more further aroma substances.

The formulation was prepared for various aromas. 5 kg of the spray blend were produced for small scale testing using a Niro Minor laboratory spray tower. Mixing was carried out for 20 min. with an Ultra Turrax. The temperature of the blend rose from 20°C to 35°C. 100 kg of the spray blend were produced for the scale-up. Over the 20 minutes of simultaneous mixing with a Conti TDS and an Ystral batch mixer, the temperature rose from 20°C to 62°C.

Spray drying was begun immediately after mixing (inlet temperature 200°C, outlet temperature 80°C). Crystal growth was characterized in terms of how spraying proceeded. Trouble-free spraying was indicated with a "No" in the crystal growth cell, while "Yes" was entered for atomization disrupted due to crystal growth. Nozzle diameter was 1 mm both in the small scale testing and the scale-up.

| Aroma | Crystal growth in small scale testing (5 kg batch) | Crystal growth in scale-up (100 kg batch) without crystal growth inhibitor | Crystal growth in scale-up (100 kg batch) with crystal growth inhibitor d-limonene |
|---|---|---|---|
| Orange* | No | No | No |
| Strawberry* | No | No | No |
| Peppermint* | No | No | No |
| Chicken | No | Yes | No |
| Beef | No | Yes | No |
| Roast potato | No | Yes | No |

| | | | |
|---|---|---|---|
| * Comparative Example | | | |

| Aroma | D(v, 0.9) in 100 kg batch without crystal growth inhibitor | D(v, 0.9) in 100 kg batch with crystal growth-inhibitor d-limonene |
|---|---|---|
| Chicken | 141.0 µm | 48.1 µm |
| Beef | 180.2 µm | 23.6 µm |
| Roast potato | 146.1 µm | 27.2 µm |

Spraying was trouble-free in every case in the small scale testing. Aromas which contain no crystal growth-promoting aroma substances (orange, strawberry, peppermint) are also unproblematic at the larger scale. However, on scale-up, disrupted spraying was observed in the case of aromas made up of crystal growth-promoting aroma substances (chicken, beef, roast potato). Some of the crystals which had led to clogging of the spray nozzle were even of a size of approx. 500 µm (measured by microscope). Crystal growth could be greatly reduced by addition of d-limonene and spraying proceeded without disruption.

### Example 2

The tests with the roast potato aroma from Example 1 were repeated, but using oleic acid as the crystal growth inhibitor instead of d-limonene. Oleic acid, like d-limonene, proved to be a very good crystal growth inhibitor.

### Example 3

The tests with the roast potato aroma from Example 1 were repeated, but using I-limonene as the crystal growth inhibitor instead of d-limonene. L-limonene may likewise be used as a very good crystal growth inhibitor.

### Example 4 (laboratory test):

The formulation from Example 1 was used to produce a small quantity of 100 g of spray blend, a laboratory Ultra-Turrax being used and mixing being carried out for 20 min.. Beta-cyclodextrin with a particle size of less than 125 µm was used. 10 g of the spray blend were placed in a sealable test tube and heated to 60°C in a water bath for 30 min.. Slow cooling was then carried out on a shaker. The roast potato aroma from test 1 was used. It was observed under a microscope whether crystals with a particle size of distinctly greater than 125 µm had formed.

| | Crystal growth | Flavor threshold value ppb | Molecular weight g/mol |
|---|---|---|---|
| d-Limonene | No | 210 | 136 |
| l-Limonene | No | 500 | 136 |
| alpha-Pinene | No | 6 | 136 |
| delta-Carene | No | 156 | 136 |
| L-Menthyl acetate | Yes | 800 | 198 |

Terpenes with the empirical formula C₁₀H₁₆ act as effective crystal growth inhibitors. Due to their elevated flavor threshold value, d-limonene and I-limonene have no influence on the aroma, delta-carene is slightly perceptible and alpha-pinene is distinctly organoleptically perceptible due to its relatively strong intrinsic flavor.

### Example 5 (required quantities of limonene):

Spray blend [wt.%]:

| | |
|---|---|
| 68.9 | Water |
| 28 | beta-Cyclodextrin |
| 2.8 | Roast potato aroma from Example 1 |
| X | d-Limonene |

| Quantity of oleic acid X [wt.%] | Effect |
|---|---|
| 0.000 | Formation of numerous large crystals |
| 0.028 | Formation of numerous large crystals |
| 0.14 | Better than before, but still not satisfactory |
| 0.28 | OK |

Quantity of Effect limonene X [wt.%] 0.000 Formation of numerous large crystals 0.028 Formation of numerous large crystals 0.14 Better than before, but still not satisfactory 0.28 OK

### Example 6 (required quantities of oleic acid):

The formulation from Example 5 is used, but using oleic acid as the crystal growth inhibitor instead of limonene.

| Quantity of oleic acid X [wt.%] | Effect |
|---|---|
| 0.000 | Formation of numerous large crystals |
| 0.014 | Formation of numerous large crystals |
| 0.07 | Better than before, but still not satisfactory |
| 0.14 | OK |

When used in a smaller quantity, oleic acid achieves the same effect as limonene. Thanks to the unbranched, elongate molecular structure, several cyclodextrin molecules can form an inclusion compound with the oleic acid molecule. Due to its elevated flavor threshold value of 2200 ppb, oleic acid is not organoleptically perceptible in the aroma.

### Example 7

Spray blend [wt.%]:

| | |
|---|---|
| 68.3 | Water |
| 28.0 | beta-Cyclodextrin |
| 2.8 | Roast potato aroma from Example 1 |
| 0.6 | Carboxymethylcellulose (Walocel CRT 10000 GA, Wolff) |
| 0.28 | d-Limonene |

The particles were produced as in Example 1. Production was unproblematic in the 100 kg batch.

### Example 8

Example 7 is repeated with 100 kg of the spray blend being dried and granulated without problems by fluidized bed spray granulation.

### Example 9

Spray blend [wt.%]:

| | |
|---|---|
| 61.2 | Water |
| 20.0 | beta-Cyclodextrin |
| 3.5 | Roast potato aroma from Example 1 |
| 15.0 | Maltodextrin DE 20 |
| 0.28 | d-Limonene |

Example of a spray blend with a solids content-increasing agent

### Example 10:

Spray blend [wt.%]:

| | |
|---|---|
| 61.2 | Water |
| 20.0 | beta-Cyclodextrin |
| 3.5 | Roast potato aroma from Example 1 |
| 15.0 | Capsul (National Starch) |
| 0.28 | d-Limonene |

Example of a spray blend with emulsifier

### Examples 11-13 (combinations of crystal growth inhibitors) :

Spray blend [wt.%]:

| | |
|---|---|
| 68.9 | Water |
| 28 | beta-Cyclodextrin |
| 2.8 | Roast potato aroma from Example 1 |
| X | d-Limonene |
| Y | Oleic acid |

The following applies with regard to X and Y:

| Example | Proportion of X (d-limonene) | Proportion of Y (oleic acid) |
|---|---|---|
| 11 | 0.07 | 0.1 |
| 12 | 0.14 | 0.07 |
| 13 | 0.2 | 0.04 |

The missing percentage is in each case made up to 100% with water.

## Claims

1. Use
(i) of a terpene of the empirical formula C₁₀H₁₆ and/or
(ii) of a mono- or polyunsaturated linear fatty acid having 16 to 18 carbon atoms
as a crystal growth inhibitor for encapsulating aroma substances in cyclodextrine particles.

2. Aroma particles comprising or consisting of
a) 70-99.3 wt.% of a cyclodextrin,
b) 0.001-20 wt.% of a crystal growth-promoting aroma substance selected from the group consisting of 2-methyl-3-(methylthio)furan, 2-methyl-3-furanthiol, bis(2-methyl-3-furyl) disulfide, furfurylmercaptan, methional, 2-acetyl-2-thiazoline, 3-mercapto-2-pentanone, 2,5-dimethyl-3-furanthiol, 2,4,5-trimethylthiazole, 2-acetylthiazole, 2,4-dimethyl-5-ethylthiazole, mercapto-3-methyl-1-butanol, 2-acetyl-1-pyrroline, 2-methyl-3-ethylpyrazine, 2-ethyl-3,5-dimethylpyrazine, 2-ethyl-3,6-dimethylpyrazine, 2,3-diethyl-5-methylpyrazine, 3-isopropyl-2-methoxypyrazine, 3-isobutyl-2-methoxypyrazine, 2-acetylpyrazine, 2-pentylpyridine, (E,E)-2,4-decadienal, (E,E)-2,4-nonadienal, (E)-2-octenal, (E)-2-nonenal, 2-undecenal, 12-methyltridecanal, 1-penten-3-one, 2,3-butanedione, 4-hydroxy-2,5-dimethyl-3(2H)-furanone, guaiacol, 3-hydroxy-4,5-dimethyl-2(5H)-furanone, 3-hydroxy-4-methyl-5-ethyl-2(5H)-furanone, dimethyl sulfide and trimethylamine, or a blend of two or more of these aroma substances,
c) 0.1-2 wt.% of crystal growth inhibitor selected from the group consisting of
(i) terpenes of the empirical formula C₁₀H₁₆ and
(ii) mono- or polyunsaturated linear fatty acids having 16 to 18 carbon atoms,
in each case relative to the total weight of the aroma particle.

3. Aroma particles according to claim 2 furthermore comprising
d) 0.5-14 wt.% of water, and
e) 0-14 wt.% of an aroma solvent,
in each case relative to the total weight of the aroma particle.

4. Spray blend comprising or consisting of
a) 5-40 wt.% of a cyclodextrin,
b) 0.0001-8 wt.-% of a crystal growth-promoting aroma substance selected from the group consisting of 2-methyl-3-(methylthio)furan, 2-methyl-3-furanthiol, bis(2-methyl-3-furyl) disulfide, furfurylmercaptan, methional, 2-acetyl-2-thiazoline, 3-mercapto-2-pentanone, 2,5-dimethyl-3-furanthiol, 2,4,5-trimethylthiazole, 2-acetylthiazole, 2,4-dimethyl-5-ethylthiazole, mercapto-3-methyl-1-butanol, 2-acetyl-1-pyrroline, 2-methyl-3-ethylpyrazine, 2-ethyl-3,5-dimethylpyrazine, 2-ethyl-3,6-dimethylpyrazine, 2,3-diethyl-5-methylpyrazine, 3-isopropyl-2-methoxypyrazine, 3-isobutyl-2-methoxypyrazine, 2-acetylpyrazine, 2-pentylpyridine, (E,E)-2,4-decadienal, (E,E)-2,4-nonadienal, (E)-2-octenal, (E)-2-nonenal, 2-undecenal, 12-methyltridecanal, 1-penten-3-one, 2,3-butanedione, 4-hydroxy-2,5-dimethyl-3(2H)-furanone, guaiacol, 3-hydroxy-4,5-dimethyl-2(5H)-furanone, 3-hydroxy-4-methyl-5-ethyl-2(5H)-furanone, dimethyl sulfide and trimethylamine, or a blend of two or more of these aroma substances,
c) 0.07-1.5 wt.% of crystal growth inhibitor selected from the group consisting of
(i) terpenes of the empirical formula C₁₀H₁₆ and
(ii) mono- or polyunsaturated linear fatty acids having 16 to 18 carbon atoms,
d) 60-94.8 wt.% of water, and
e) 0-6 wt.% of an aroma solvent
in each case relative to the total weight of the aroma particle.

5. A method for producing aroma particles comprising spraying a spray blend according to claim 4 through an atomizer.

6. A method according to claim 5, **characterized in that** the spray blend is dried in an atomization drying method.

7. A method according to claim 5 or claim 6, **characterized in that** at least 50 l of the spray blend per atomizer are continuously spray dried.

## Patentansprüche

1. Verwendung
(i) eines Terpens der empirischen Formel C₁₀H₁₆ und/oder
(ii) einer einfach oder mehrfach ungesättigten linearen Fettsäure mit 16 bis 18 Kohlenstoffatomen
als Kristallwachstumsinhibitor zum Verkapseln von Aromastoffen in Cyclodextrinpartikeln.

2. Aromapartikel, umfassend oder bestehend aus
a) 70-99,3 Gew.-% eines Cyclodextrins,
b) 0,001-20 Gew.-% eines das Kristallwachstum fördernden Aromastoffes ausgewählt aus der Gruppe bestehend aus 2-Methyl-3-(methylthio)furan, 2-Methyl-3-furanthiol, Bis(2-methyl-3-furyl)disulfid, Furfurylmercaptan, Methional, 2-Acetyl-2-thiazolin, 3-Mercapto-2-pentanon, 2,5-Dimethyl-3-furanthiol, 2,4,5-Trimethylthiazol, 2-Acetylthiazol, 2,4-Dimethyl-5-ethylthiazol, Mercapto-3-methyl-1-butanol, 2-Acetyl-1-pyrrolin, 2-Methyl-3-ethylpyrazin, 2-Ethyl-3,5-dimethylpyrazin, 2-Ethyl-3,6-dimethylpyrazin, 2,3-Diethyl-5-methylpyrazin, 3-Isopropyl-2-methoxypyrazin, 3-Isobutyl-2-methoxypyrazin, 2-Acetylpyrazin, 2-Pentylpyridin, (E,E)-2,4-decadienal, (E,E)-2,4-nonadienal, (E)-2-octenal, (E)-2-nonenal, 2-Undecenal, 12-Methyltridecanal, 1-Penten-3-on, 2,3-Butandion, 4-Hydroxy-2,5-dimethyl-3(2H)-furanon, Guaiacol, 3-Hydroxy-4,5-dimethyl-2(5H)-furanon, 3-Hydroxy-4-methyl-5-ethyl-2(5H)-furanon, Dimethylsulfid und Trimethylamin, oder einer Mischung von zwei oder mehr dieser Aromastoffe,
c) 0,1-2 Gew.-% eines Kristallwachstumsinhibitors, ausgewählt aus der Gruppe bestehend aus
(i) Terpenen der empirischen Formel C₁₀H₁₆ und
(ii) einfach oder mehrfach ungesättigten linearen Fettsäuren mit 16 bis 18 Kohlenstoffatomen,
in jedem Fall bezogen auf das Gesamtgewicht des Aromapartikels.

3. Aromapartikel nach Anspruch 2, weiter umfassend:
d) 0,5-14 Gew.-% Wasser, und
e) 0-14 Gew.-% eines Aromalösungsmittels,
in jedem Fall bezogen auf das Gesamtgewicht des Aromapartikels.

4. Sprühmischung, umfassend oder bestehend aus
a) 5-40 Gew.-% eines Cyclodextrins,
b) 0,0001-8 Gew.-% eines das Kristallwachstum fördernden Aromastoffes ausgewählt aus der Gruppe bestehend aus 2-Methyl-3-(methylthio)furan, 2-Methyl-3-furanthiol, Bis(2-methyl-3-furyl)disulfid, Furfurylmercaptan, Methional, 2-Acetyl-2-thiazolin, 3-Mercapto-2-pentanon, 2,5-Dimethyl-3-furanthiol, 2,4,5-Trimethylthiazol, 2-Acetylthiazol, 2,4-Dimethyl-5-ethylthiazol, Mercapto-3-methyl-1-butanol, 2-Acetyl-1-pyrrolin, 2-Methyl-3-ethylpyrazin, 2-Ethyl-3,5-dimethylpyrazin, 2-Ethyl-3,6-dimethylpyrazin, 2,3-Diethyl-5-methylpyrazin, 3-Isopropyl-2-methoxypyrazin, 3-Isobutyl-2-methoxypyrazin, 2-Acetylpyrazin, 2-Pentylpyridin, (E,E)-2,4-decadienal, (E,E)-2,4-nonadienal, (E)-2-octenal, (E)-2-nonenal, 2-Undecenal, 12-Methyltridecanal, 1-Penten-3-on, 2,3-Butandion, 4-Hydroxy-2,5-dimethyl-3(2H)-furanon, Guaiacol, 3-Hydroxy-4,5-dimethyl-2(5H)-furanon, 3-Hydroxy-4-methyl-5-ethyl-2(5H)-furanon, Dimethylsulfid und Trimethylamin, oder einer Mischung von zwei oder mehr dieser Aromastoffe,
c) 0,07-1,5 Gew.-% eines Kristallwachstumsinhibitors, ausgewählt aus der Gruppe bestehend aus
(i) Terpenen der empirischen Formel C₁₀H₁₆ und
(ii) einfach oder mehrfach ungesättigten linearen Fettsäuren mit 16 bis 18 Kohlenstoffatomen,
d) 60-94,8 Gew.-% Wasser, und
e) 0-6 Gew.-% eines Aromalösungsmittels,
in jedem Fall bezogen auf das Gesamtgewicht des Aromapartikels.

5. Verfahren zur Herstellung von Aromapartikeln, umfassend das Versprühen einer Sprühmischung nach Anspruch 4 durch einen Zerstäuber.

6. Verfahren nach Anspruch 5, **dadurch gekennzeichnet, dass** die Sprühmischung in einem Zerstäubungstrocknungsverfahren getrocknet wird.

7. Verfahren nach Anspruch 5 oder Anspruch 6, **dadurch gekennzeichnet, dass** mindestens 50 l der Sprühmischung pro Zerstäuber kontinuierlich sprühgetrocknet werden.

## Revendications

1. Utilisation:
(i) d'un terpène de la formule empirique C₁₀H₁₆ et/ou
(ii) d'un acide gras linéaire mono- ou polyinsaturé possédant de 16 à 18 atomes de carbone
en tant qu'inhibiteur de croissance de cristaux pour l'encapsulation de substances d'arôme dans des particules de cyclodextrine.

2. Particules d'arôme comprenant ou constituées de:
a) 70-99,3% en poids d'une cyclodextrine,
b) 0,001-20% en poids d'une substance d'arôme favorisant la croissance de cristaux choisie dans le groupe constitué de 2-méthyl-3-(méthylthio)furane, de 2-méthyl-3-furanethiol, de disulfure de bis(2-méthyl-3-furyle), de furfurylmercaptan, de méthional, de 2-acétyl-2-thiazoline, de 3-mercapto-2-pentanone, de 2,5-diméthyl-3-furanethiol, de 2,4,5-triméthylthiazole, de 2-acétylthiazole, de 2,4-diméthyl-5-éthylthiazole, de mercapto-3-méthyl-1-butanol, de 2-acétyl-1-pyrroline, de 2-méthyl-3-éthylpyrazine, de 2-éthyl-3,5-diméthylpyrazine, de 2-éthyl-3,6-diméthylpyrazine, de 2,3-diéthyl-5-méthylpyrazine, de 3-isopropyl-2-méthoxypyrazine, de 3-isobutyl-2-méthoxypyrazine, de 2-acétylpyrazine, de 2-pentylpyridine, de (E,E)-2,4-décadiénal, de (E,E)-2,4-nonadiénal, de (E)-2-octénal, de (E)-2-nonénal, de 2-undécénal, de 12-méthyltridécanal, de 1-pentén-3-one, de 2,3-butanedione, de 4-hydroxy-2,5-diméthyl-3(2H)-furanone, de guaïacol, de 3-hydroxy-4,5-diméthyl-2(5H)-furanone, de 3-hydroxy-4-méthyl-5-éthyl-2(5H)-furanone, de sulfure de diméthyle et de triméthylamine ou d'un mélange de deux ou plus de ces substances d'arôme,
c) 0,1-2% en poids d'inhibiteur de croissance de cristaux choisi dans le groupe constitué:
(i) de terpènes de la formule empirique C₁₀H₁₆ et
(ii) d'acides gras linéaires mono- ou polyinsaturés possédant de 16 à 18 atomes de carbone,
dans chaque cas relativement au poids total de la particule d'arôme.

3. Particules d'arôme selon la revendication 2, comprenant en outre:
d) 0,5-14% en poids d'eau, et
e) 0-14% en poids d'un solvant d'arôme,
dans chaque cas relativement au poids total de la particule d'arôme.

4. Mélange de pulvérisation comprenant ou constitué de:
a) 5-40% en poids d'une cyclodextrine,
b) 0,0001-8% en poids d'une substance d'arôme favorisant la croissance de cristaux choisie dans le groupe constitué de 2-méthyl-3-(méthylthio)furane, de 2-méthyl-3-furanethiol, de disulfure de bis(2-méthyl-3-furyle), de furfurylmercaptan, de méthional, de 2-acétyl-2-thiazoline, de 3-mercapto-2-pentanone, de 2,5-diméthyl-3-furanethiol, de 2,4,5-triméthylthiazole, de 2-acétylthiazole, de 2,4-diméthyl-5-éthylthiazole, de mercapto-3-méthyl-1-butanol, de 2-acétyl-1-pyrroline, de 2-méthyl-3-éthylpyrazine, de 2-éthyl-3,5-diméthylpyrazine, de 2-éthyl-3,6-diméthylpyrazine, de 2,3-diéthyl-5-méthylpyrazine, de 3-isopropyl-2-méthoxypyrazine, de 3-isobutyl-2-méthoxypyrazine, de 2-acétylpyrazine, de 2-pentylpyridine, de (E,E)-2,4-décadiénal, de (E,E)-2,4-nonadiénal, de (E)-2-octénal, de (E)-2-nonénal, de 2-undécénal, de 12-méthyltridécanal, de 1-pentén-3-one, de 2,3-butanedione, de 4-hydroxy-2,5-diméthyl-3(2H)-furanone, de guaïacol, de 3-hydroxy-4,5-diméthyl-2(5H)-furanone, de 3-hydroxy-4-méthyl-5-éthyl-2(5H)-furanone, de sulfure de diméthyle et de triméthylamine ou d'un mélange de deux ou plus de ces substances d'arôme,
c) 0,07-1,5% en poids d'inhibiteur de croissance de cristaux choisi dans le groupe constitué:
(i) de terpènes de la formule empirique C₁₀H₁₆ et
(ii) d'acides gras linéaires mono- ou polyinsaturés possédant de 16 à 18 atomes de carbone,
d) 60-94,8% en poids d'eau, et
e) 0-6% en poids d'un solvant d'arôme,
dans chaque cas relativement au poids total de la particule d'arôme.

5. Procédé pour la production de particules d'arôme comprenant la pulvérisation d'un mélange de pulvérisation selon la revendication 4 par un atomiseur.

6. Procédé selon la revendication 5, **caractérisé en ce que** le mélange de pulvérisation est séché dans un procédé de séchage par atomisation.

7. Procédé selon la revendication 5 ou la revendication 6, **caractérisé en ce que** au moins 50 1 du mélange de pulvérisation par atomiseur sont séchés par atomisation en continu.
